# EUROPEAN PATENT SPECIFICATION

(11) **EP 2 257 261 B1**
(45) Date of publication and mention of the grant of the patent: **07.10.2015**
(21) Application number: 09713462.1
(22) Date of filing: 20.02.2009
(51) Int. Cl.: A61J 1/20, A61M 5/315, A61M 5/20, A61M 5/24

(54) **DISPENSER FOR LOCAL ANAESTHETICS AND OTHER LIQUIDS**
SPENDER FÜR LOKALANÄSTHETIKA UND ANDERE FLÜSSIGKEITEN
DISTRIBUTEUR POUR ANESTHÉSIQUES LOCAUX ET AUTRES LIQUIDES

(30) Priority: 21.02.2008 DK 200800244
(43) Date of publication of application: 08.12.2010
(73) Proprietor: Nielsen, Poul Torben, 9200 Åalborg SV (DK)
(72) Inventor: Nielsen, Poul Torben, 9200 Åalborg SV (DK)
(74) Representative: Nielsen, Leif
(86) International application number: PCT/DK2009/000049
(87) International publication number: WO 2009/103297

(56) References cited:
- CA-A1- 2 295 249
- GB-A- 1 111 382
- GB-A- 1 174 936
- SU-A1- 793 580
- US-A- 3 827 601

## Description

### Field of the Invention

The present invention concerns a device for transferring local anaesthetics from a reservoir to a hypodermic needle or a catheter which device includes a well-defined pump chamber in a pump housing which is provided with a manually displaceable pump piston which at one end outside the pump housing is provided with a pump grip, wherein the pump housing at an inlet has a first connection element for joining with a corresponding first connection element on the outlet of the reservoir, and wherein the pump housing has a second connection element at an outlet for joining with a corresponding second connection element on the needle or the catheter, and wherein the pump piston is connected via a piston rod with the pump grip which is pivotably suspended by joints, and which is actuated for transferring liquid from the reservoir to the hypodermic needle or the catheter for introducing the liquid into the tissue. The device is intended for use in the dispensing of local anaesthetics from the reservoir via the hypodermic needle or the catheter to tissue, subcutis, muscles, fascia, joint capsule, bone or articular cavity.

### Background of the Invention

Pain after surgical interventions frequently require administration of painkillers, in particular the first hours/days after the operation. The traditional administration forms are orally as tablets, parenterally, intramuscularly, epidurally and by conduction anaesthesia.

In recent years, so-called local application or infiltration of local anaesthetic in the surgical field has found increasing use after large surgical interventions. This kind of administration does not imply the side effects which the above mentioned traditional forms of administration imply, including i.a. nausea, respiration depression, blunted sensorium and affected motor function, and many others. Besides that these unfavourable side effects do not occur by the new administration form/local infiltration, this method also allows for rapid mobilisation, i.e. few hours after comprehensive joint operations, and a possibility of an earlier discharge.

By the hitherto used method of local infiltration or application of local anaesthetics after larger surgical interventions, typically conventional hypodermic syringes with a volume of 20-100 ml and conventional hypodermic needles for transferring the local anaesthetic in fluid form from a reservoir, which e.g. can be polyethylene bag, a plastic bottle, a glass bottle or similar, via the hypodermic needle to tissue, skin, subcutis, muscles, fascia, joint capsule, bone or articular cavity. This procedure implies a risk of sterility failure and jab accidents, and is time-consuming in addition.

If large hypodermic syringes (50-100ml) are used, a significantly greater force is required for driving the liquid out from the syringe to the needles and out into the tissue. Besides, there is no possibility of a more accurate dosing, as the driving out of the liquid occurs continually, and the dosing flow (measured as volume per second) depends on the applied force, the diameter of the used syringe, the length and inner diameter of the used needle, and finally of the resistance offered by the tissue.

GB 1174936 and GB 1111382 disclose a device mentioned by way of introduction. This device provides a pump device is disposed between the hypodermic needle or catheter and the reservoir. The device is used for animal, preferably sheep in order to introduce medicaments in form of liquid or paste into the animal by a positively operated plunger. There is no disclosure of a safety function if the hypodermic needle has been put in an unsuitable place where the fluid resistance impedes liquid transfer into the animal.

The drawbacks of the prior art procedure solved by the invention include sterility failure, jab accidents, inaccurate dosing, great exertion of force by dosing and a time-consuming procedure.

### Object of the Invention

On this background, it is the object of the present invention to indicate a device enabling transfer of liquids, e.g. local anaesthetics, from the reservoir via the hypodermic needle or the catheter to the tissue, skin, subcutis, muscles, fascia, joint capsule, bone or the articular cavity in a safe way without the disadvantages associated with the prior art procedure.

### Description of the Invention

According to the present invention, this object is achieved by a device of the kind mentioned in the introduction, which is peculiar in that the pump grip and the joints are provided with a friction lining thereby providing a friction coupling it includes a well-defined pump chamber in a pump housing which is provided with a manually displaceable pump piston which at one end outside the pump housing is provided with a pump grip, that the pump housing at an inlet has a first connection element for joining with a corresponding first connection element on the outlet of the reservoir , and that the pump housing has a second connection element at an outlet for joining with a corresponding second connection element on the needle or the catheter.

The greatest advantages achieved by the invention are the possibility of a very precise and safe dosing by using the dispenser, and that an otherwise time-consuming procedure is simplified such that this procedure can be performed much quicker than it otherwise would.

The friction coupling provides a safety function with regard to wrong disposition of the hypodermic needle during transfer of the liquid. If the hypodermic needle has been put in an unsuitable place where the fluid resistance impedes liquid transfer, the friction coupling will entail that injury/damage will occur neither on patient nor on device/apparatus.

Moreover there is achieved a reduction of sterility failure, jab accidents, inaccurate dosing, great exertion of force by dosing and a time-consuming procedure. The pump grip thus enables a more certain dosing of the liquid directly from the reservoir via the pump to hypodermic needle/the catheter to tissue, subcutis, muscles, fascia, joint capsule, bone or articular cavity. There is no need of a separate procedure for drawing the liquid to the needle and a subsequent procedure for introducing the liquid into housing or the like.

Moreover the device makes it possible to achieve flexibility in relation to the position of reservoir and patient and the other surroundings such that the positioning of the device with pivotable handle-operated pump can be regulated with the intention of optimising the position for optimal effect and the least possible nuisance for patient and surroundings. The pivoting handle is articulated on the device such that a lever action is established; contributing to reduced exertion of force, and simultaneously pivoting through a larger or smaller angle may be provided at the same time for increasing the precision of the injected amount of liquid. A device where a larger pivoting angle is established will enable a certain dosing, since a smaller volume is discharged by pivoting the pump grip through a given angle.

The device is furthermore characterised in that the pump piston is formed by a piston crown and the piston rod, and that the pump chamber is formed between the piston crown on the pump piston and the outlet of the pump housing. When transferring liquid from the reservoir to the hypodermic needle or a catheter for introducing into tissue, a well-defined volume can be established in the pump chamber. Particularly if the pump is of a type with longitudinal adjustment of the stroke of the pump piston, there may be attained a well-defined amount in each pump stroke in a simple way.

Hereby it is moreover possible to reduce the large use of force usually required in connection with this procedure, as a manually operated pump device with a well-defined pump chamber reduces the complexity of the transfer of liquid from a reservoir to a hypodermic needle or a catheter for introducing into tissue or a cavity between the needle or catheter and the reservoir.

The device is furthermore characterised in that the first connection element is provided in the form of a pointed cannula. The pointed cannula may penetrate a traditional membrane in reservoirs in the form of glass bottles and conduct the liquid from the reservoir to the pump device.

Hereby is achieved a reduction of the risk of jab accidents and sterility failure, as the point is passed through the membrane of the reservoir and is disposed inside the reservoir and remains in this position during the transfer of liquid from the reservoir to the destination of the liquid.

The device may furthermore be characterised in that it is constituted by a disposable unit in which reservoir and pump are integrated.

Hereby is achieved further optimisation of reduction of the parameters jab accident and sterility failure, as by this embodiment all unnecessary joints of the device are eliminated. All unneeded connection elements are removed from this embodiment.

According to another embodiment, the device may be characterised in that a filter is provided in connection with the outlet of the reservoir. The liquid may thereby be conducted from the reservoir to the pump device via a particulate filter.

Hereby may be achieved an advantageous filtering for further reducing sterility failure and simultaneous cleaning of the liquid.

The device may furthermore be characterised in that the connection to the outlet of the reservoir is made with an air intake for substituting displaced liquid from the reservoir.

Hereby is achieved the immediate advantage that a reservoir of non-soft material can retain its original shape and thereby not be unnecessarily deformed, additionally facilitating transfer of the liquid.

The device may additionally be characterised in that a stroke adjustment is provided for setting the dosed volume of each stroke. The stroke adjustment can be established via a nut or other means on handle for setting the dosed volume of each stroke.

Hereby may be achieved that amount volume dosing becomes very precise and may be set or adjusted such that the wanted dosing is achieved, furthermore avoiding the inaccurate dosing resulting from the conventional method.

The device may furthermore be characterised in that the pump grip is provided with recesses for finger grip.

Hereby is achieved a more firm grip around the handle such that it does not easily slide out of proper position, and this will thus also enhance the precision by which the transfer of liquid occurs.

The device includes several advantageous embodiments enabling that it may be designed with the following constituent elements:
- Removable protective device around the above hypodermic needle.
- Particulate filter.
- Locking mechanism (snap-lock, screw thread, bayonet socket etc.) keeping the dispenser fixed on the reservoir.
- Reservoir.
- Packing safeguarding against leakage of liquid between the hypodermic needle and the reservoir.
- Pump device (piston, membrane etc.).
- Handle for actuating the pump device ("trigger").
- Connection between pump and adapter.
- Adapter for fixing hypodermic needle (screw thread, Leur-lock, conical adaptation etc.).

The dispenser may alternatively be designed such that the dispenser device itself (pointed cannula from reservoir, possibly particulate filter, pump housing and pump device and adapter for hypodermic needle) are built together with the reservoir itself, whether the case is a polyethylene bag, a plastic bottle, a glass bottle or the like. If reservoir and dispenser device are integrated or built together, it is equipment for single use.

### Description of use of the dispenser

The dispenser is removed from the sterile packing. The protective cover or cap over the cannula (which is to be inserted in the reservoir) is removed. The cannula is then moved against the central part of the membrane in the reservoir and is brought to penetrate the membrane and introduced in the reservoir. The insertion is finished when there is contact between the membrane in the reservoir and the packing provided in the dispenser around the cannula. Tightening/locking is achieved by actuating the locking mechanism (collar with screw thread, snap-lock or similar). Suitable hypodermic needle is then provided with Leur-lock, screw thread or conical adapter, and this is fixed to the adapter on the dispenser. The protective cap over the hypodermic needle is then removed, after which the device is ready for use. The liquid, frequently local anaesthetic, may then be distributed in tissue and articular cavities by repeated actuations of the pump and by pre-determined and well-defined doses (volume) each time the pump is actuated. When infusion of the liquid is completed, the protective cap is put on the hypodermic needle, and the device may then be destroyed without performing any kind of separation between reservoir and dispenser.

Alternatively, the dispenser can be built together with the reservoir, whereby further reduction of jab accidents and sterility failure is achieved.

### Material

The dispenser (and possibly integrated reservoir) can be made of all kinds of solid materials, including metal and plastic as well. The device is mainly intended for single use and then preferably of plastic material.

### Dimensions

The locking or fixing mechanism between the dispenser and the reservoir is made with dimensions which can be adapted to the membrane capsules and other propane devices on polyethylene bags, glass bottles, plastic bottles and other reservoir devices.

The adapter (or "stub") on the dispenser is made with dimensions adapted to the hypodermic needles available on the market.

The pump device is dimensioned such that by each pump stroke, a well-defined volume (typically 0.1 - 1 ml) of liquid is conducted from the reservoir via the dispenser to the hypodermic needle.

### Description of the Drawing

The invention is explained in more detail with reference to the drawings, in which the design of the dispenser is seen sketched, and in which:
- Fig. 1: illustrates a first embodiment of the invention, where the reservoir is disposed with the opening down over the hypodermic needle, and where the pump grip is provided in a vertically upwards directed position;
- Fig. 2: illustrates a second embodiment of the invention, where the reservoir is disposed below the hypodermic needle and the pump grip is provided in a vertically downwards directed position;
- Fig. 3: illustrates a third embodiment of the invention, where the pump grip is provided in a vertically downwards directed position; and
- Fig. 4: illustrates a fourth embodiment of the invention, where the reservoir is disposed in continuation of the hypodermic needle in a horizontal position, and the pump grip is provided in a downwards directed, vertical position. In this embodiment, there is furthermore provided a handle for supporting the pump grip.

### Detailed Description of the Invention

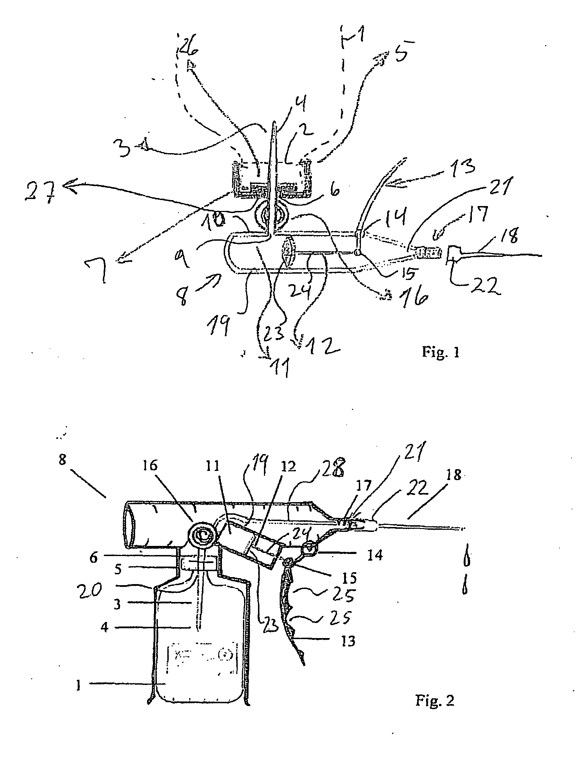

On Fig. 1, a broken line shows schematically a reservoir 1 in the form of a glass bottle with a membrane 2 which is penetrated by a sharp pointed cannula 3, where the innermost part of the point has an opening 4 for discharging liquid from the reservoir 1.

The reservoir 1 is provided an outlet 20 at the neck opening of the bottle. There is provided a connection element 5 with a hole 6 through which the pointed cannula 3 extends. The connection element 5 can be a snap-on cover, a cover with screw thread or possibly a Leur-lock. In any case, this serves as connection to the reservoir 1 in a sealed way. In order to enhance the sealing, a packing 26 may be provided inside the connection element/cover 5. Moreover, together with the packing there may be provided a further sealing for the cannula 4 in the form of a collar 7.

The connection to the outlet 6 of the reservoir is made with an air intake 27 for substituting displaced liquid from the reservoir 1. The air intake is designed as a non-return valve that only allows air to penetrate into the reservoir for replacing the displaced liquid.

The pointed cannula 3 is connected to a pump device 8 as it passes through an opening in a sidewall 10 on the pump housing 19 of the pump device. The opening constitutes the inlet 9 of the pump housing. Within the pump housing a pump piston 12 is provided having a piston crown 23 and a piston rod 24 which is connected with a pump grip 13 hinged about an articulated joint 14 and articulated connected with the piston rod 24 of the pump piston 12 via an articulated joint 15. Hereby may be formed a lever action providing that only limited force is to be applied in order to operate the pump device.

The pointed cannula 3 can be provided with a filter 16 such that particles are not drawn into the pump device 8.

The pump device 8 is provided with a second connection element 17 in the form of an adapter intended for connection with a second connection element 22 on a hypodermic needle which is schematically illustrated with the reference number 18. Alternatively, a catheter with such a second connection element can be used. On Fig. 2 is illustrated a second embodiment of the invention, where the reservoir 1 is disposed below the pointed cannula 3, and the pump grip 13 is provided in a vertically downwards directed position. Here, the pump device is of a type that sucks the liquid up from the reservoir 1 and presses the liquid from the pump housing 19 through a conduit 28, the outer end of which constituting an outlet 21 from the pump housing 19. Moreover, the adapter 17 is seen connected to a hypodermic needle 18, and the pump grip 13 is connected to the articulated joint 15 which is directly connected to the pump piston 12 and furthermore connected to the articulated joint 14. Moreover, the pump grip is provided with recesses 25 where fingers may engage without risk of slipping.

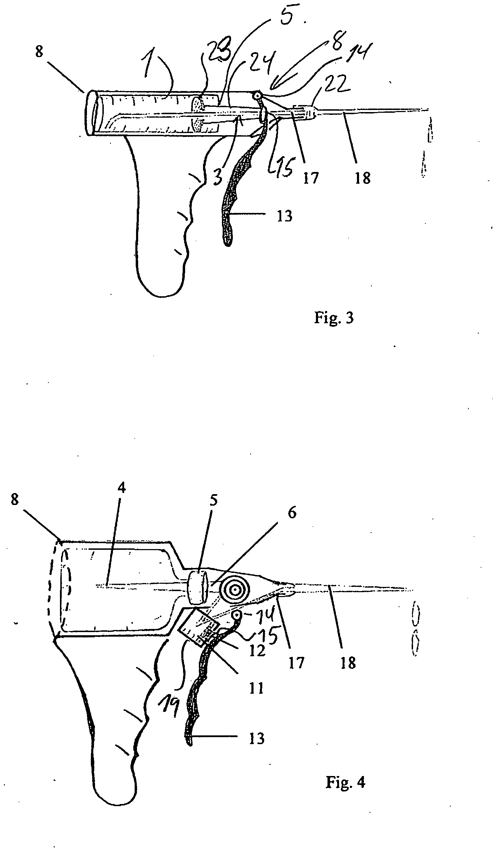

On Fig. 3 is illustrated a third embodiment of the invention, where the pump grip 13 is provided in a vertically downwards directed position.

On Fig. 4 is illustrated a fourth embodiment of the invention, where the reservoir 1 is disposed in continuation of the pointed cannula 3 in a horizontal position, and the pump grip 13 is provided in a downwards directed, vertical position. In this embodiment, there is furthermore provided a holder for supporting the pump grip.

The above illustrations are just schematic embodiments of a device according to the invention. The device may be designed in other ways than shown above when only it is possible to perform a connection with a pump device which is disposed between the hypodermic needle 18 and the reservoir 1.

### Overview

1 - reservoir
2 - membrane
3 - first connection element/pointed cannula on pump housing
4 - opening in cannula
5 - corresponding first connection element on reservoir (snap-on cover, a cover with screw thread or possibly a Leur-lock)
6 - hole/opening (reservoir outlet)
7 - collar
8 - pump device
9 - inlet/opening of pump housing
10 - sidewall
11 - pump chamber
12 - pump piston
13 - pump grip/ handgrip / operating means
14 - articulated joint
15 - articulated joint
16 - filter
17 - adapter/second connection element on pump housing
18 - hypodermic needle
19 - pump housing
20 - outlet of reservoir
21 - outlet of pump housing
22 - second connection element on hypodermic needle or catheter
23 - piston crown
24 - piston rod
25 - recesses for finger grip
26 - packing
27 - air intake
28 - conduit

## Claims

1. A device for transferring local anaesthetics from a reservoir to a hypodermic needle or catheter, which device includes a well-defined pump chamber (11) in a pump housing (19) which is provided with a manually displaceable pump piston (12) which at one end outside the pump housing (19) is provided with a pump grip (13), wherein the pump housing (19) at an inlet (9) has a first connection element (3) for joining with a corresponding first connection element (5) on the outlet (20) of the reservoir (1), and wherein the pump housing has a second connection element (17) at an outlet (21) for joining with a corresponding second connection element (22) on the needle (18) or the catheter, and wherein the pump piston (12) is connected via a piston rod (24) with the pump grip (13) which is pivotably suspended by joints (14, 15), and which is actuated for transferring liquid from the reservoir (1) to the hypodermic needle (18) or the catheter for introducing the liquid into the tissue, **characterised in that** the pump grip (13) and the joints (14, 15) are provided with a friction lining thereby providing a friction coupling.

2. Device according to claim 1, **characterised in that** the pump piston (12) is formed by a piston crown (23) and the piston rod (24), and that the pump chamber (11) is formed between the piston crown (23) on the pump piston and the outlet (21) of the pump housing.

3. Device according to claim 1 or 2, **characterised in that** the first connection element is provided in the form of a pointed cannula (3).

4. Device according to any preceding claim, **characterised in that** it is constituted by a disposable unit in which reservoir (1) and pump (8) are integrated.

5. Device according to any preceding claim, **characterised in that** a filter (16) is provided in connection with the outlet (6) of the reservoir.

6. Device according to any preceding claim, **characterised in that** the connection to the outlet (6) of the reservoir is made with an air intake (27) for substituting displaced liquid from the reservoir (1).

7. Device according to any preceding claim, **characterised in that** a stroke adjustment is provided for setting dosed volume in each stroke.

8. Device according to any preceding claim, **characterised in that** the pump grip (13) is provided with recesses (25) for finger grip.

## Patentansprüche

1. Vorrichtung zum Übertragen von Lokalanästhetika von einem Vorratsbehälter an eine hypodermische Nadel oder einen Katheter, wobei die Vorrichtung eine wohl definierte Pumpkammer (11) in einem Pumpgehäuse (19) umfasst, die mit einem manuell verlagerbaren Pumpkolben (12) versehen ist, der an einem Ende außerhalb des Pumpgehäuses (19) mit einem Pumpgriff (13) versehen ist, wobei das Pumpgehäuse (19) an einem Einlass (9) ein erstes Verbindungselement (3) für die Verbindung mit einem entsprechenden ersten Verbindungselement (5) am Auslass (20) des Vorratsbehälters (1) besitzt und wobei das Pumpgehäuse ein zweites Verbindungselement (17) an einem Auslass (21) für die Verbindung mit einem entsprechenden zweiten Verbindungselement (22) an der Nadel (18) oder an dem Katheter besitzt und wobei der Pumpkolben (12) über eine Kolbenstange (24) mit dem Pumpgriff (13) verbunden ist, der durch Gelenke (14, 15) schwenkbar aufgehängt ist und der betätigt wird, um Flüssigkeit von dem Vorratsbehälter (1) an die hypodermische Nadel (18) oder an den Katheter zu übertragen, um die Flüssigkeit in das Gewebe einzuleiten, **dadurch gekennzeichnet, dass** der Pumpgriff (13) und die Gelenke (14, 15) mit einer Reibverkleidung versehen sind, wodurch eine Reibkopplung geschaffen wird.

2. Vorrichtung nach Anspruch 1, **dadurch gekennzeichnet, dass** der Pumpkolben (12) durch eine Kolbenkrone (23) und die Kolbenstange (24) gebildet ist und dass die Pumpkammer (11) zwischen der Kolbenkrone (23) an dem Pumpkolben und dem Auslass (21) des Pumpgehäuses gebildet ist.

3. Vorrichtung nach Anspruch 1 oder 2, **dadurch gekennzeichnet, dass** das erste Verbindungselement in Form einer zugespitzten Kanüle (3) vorgesehen ist.

4. Vorrichtung nach einem vorhergehenden Anspruch, **dadurch gekennzeichnet, dass** sie durch eine wegwerfbare Einheit gebildet ist, in die der Vorratsbehälter (1) und die Pumpe (8) integriert sind.

5. Vorrichtung nach einem vorhergehenden Anspruch, **dadurch gekennzeichnet, dass** an dem Auslass (6) des Vorratsbehälters ein damit verbundener Filter (16) vorgesehen ist.

6. Vorrichtung nach einem vorhergehenden Anspruch, **dadurch gekennzeichnet, dass** die Verbindung mit dem Auslass (6) des Vorratsbehälters mit einem Lufteinlass (27) versehen ist, um aus dem Vorratsbehälter (1) verdrängte Flüssigkeit zu ersetzen.

7. Vorrichtung nach einem vorhergehenden Anspruch, **dadurch gekennzeichnet, dass** eine Hubeinstellung vorgesehen ist, um ein abgemessenes Volumen bei jedem Hub einzustellen.

8. Vorrichtung nach einem vorhergehenden Anspruch, **dadurch gekennzeichnet, dass** der Pumpgriff (13) mit Aussparungen (25) für einen Fingergriff versehen ist.

## Revendications

1. Dispositif pour transférer des anesthésiques locaux depuis un réservoir dans une aiguille hypodermique ou un cathéter, lequel dispositif comprend une chambre de pompe bien définie (11) dans un boîtier de pompe (19) qui est doté d'un piston de pompe (12) déplaçable à la main qui à une extrémité à l'extérieur du boîtier de pompe (19) est doté d'une poignée de pompe (13), dans lequel le boîtier de pompe (19) à une admission (9) comporte un premier élément de raccordement (3) pour la jonction avec un premier élément de raccordement (5) correspondant sur la sortie (20) du réservoir (1), et dans lequel le boîtier de pompe comporte un second élément de raccordement (17) à une sortie (21) pour la jonction avec un second élément de raccordement (22) correspondant sur l'aiguille (18) ou le cathéter, et dans lequel le piston de pompe (12) est relié par l'intermédiaire d'une tige de piston (24) à la poignée de pompe (13) qui est suspendue de manière pivotante par les charnières (14, 15), et qui est actionnée pour transférer le liquide depuis le réservoir (1) dans l'aiguille hypodermique (18) ou le cathéter pour introduire le liquide dans le tissu, **caractérisé en ce que** la poignée de pompe (13) et les charnières (14, 15) sont dotées d'une garniture assurant ainsi un embrayage à friction.

2. Dispositif selon la revendication 1, **caractérisé en ce que** le piston de pompe (12) est formé par une couronne de piston (23) et la tige de piston (24), et **en ce que** la chambre de pompe (11) est formée entre la couronne de piston (23) sur le piston de pompe et la sortie (21) du boîtier de pompe.

3. Dispositif selon la revendication 1 ou 2, **caractérisé en ce que** le premier élément de raccordement est prévu dans la forme d'une canule pointue (3).

4. Dispositif selon l'une quelconque des revendications précédentes, **caractérisé en ce qu'**il est constitué d'une unité jetable dans laquelle le réservoir (1) et la pompe (8) sont intégrés.

5. Dispositif selon l'une quelconque des revendications précédentes, **caractérisé en ce qu'**un filtre (16) est relié à la sortie (6) du réservoir.

6. Dispositif selon l'une quelconque des revendications précédentes, **caractérisé en ce que** le raccordement à la sortie (6) du réservoir est effectué avec une admission d'air (27) pour remplacer le liquide déplacé depuis le réservoir (1).

7. Dispositif selon l'une quelconque des revendications précédentes, **caractérisé en ce qu'**un réglage de la course est prévu pour régler le volume dosé dans chaque course.

8. Dispositif selon l'une quelconque des revendications précédentes, **caractérisé en ce que** la poignée de pompe (13) est dotée de renfoncements (25) pour la prise des doigts.
